(19) Europäisches Patentamt
European Patent Office
Office européen des brevets

(11) Publication number: **0 171 178**
**A1**

(12) # EUROPEAN PATENT APPLICATION

(21) Application number: **85304763.7**

(22) Date of filing: **04.07.85**

(51) Int. Cl.⁴: **B 01 J 23/78**, C 07 C 29/15

(30) Priority: **07.07.84 GB 8417400**
**26.01.85 GB 8501981**
**26.01.85 GB 8501980**

(43) Date of publication of application: **12.02.86**
**Bulletin 86/7**

(84) Designated Contracting States: **BE DE FR GB IT NL**

(71) Applicant: **The British Petroleum Company p.l.c., Britannic House Moor Lane, London EC2Y 9BU (GB)**

(72) Inventor: **Ball, William John, The British Petroleum Company p.l.c. Chertsey Road, Sunbury-on-Thames Middlesex, TW16 7LN (GB)**
Inventor: **Dyke, Andrew Frank, The British Petroleum Company p.l.c. Chertsey Road, Sunbury-on-Thames Middlesex, TW16 7LN (GB)**
Inventor: **Lafferty, Joseph, The British Petroleum Company p.l.c. Chertsey Road, Sunbury-on-Thames Middlesex, TW16 7LN (GB)**
Inventor: **Walker, David William, The British Petroleum Company p.l.c. Chertsey Road, Sunbury-on-Thames Middlesex, TW16 7LN (GB)**
Inventor: **Wilcox, Michael John, The British Petroleum Company p.l.c. Chertsey Road, Sunbury-on-Thames Middlesex, TW16 7LN (GB)**

(74) Representative: **Fawcett, Richard Fennelly et al, BP INTERNATIONAL LIMITED Patents Division Chertsey Road, Sunbury-on-Thames Middlesex, TW16 7LN (GB)**

(54) **Catalyst, process for its production and its use in the conversion of syngas to methanol.**

(57) A catalyst precursor for use after reductive activation in the conversion of synthesis gas to methanol having the formula:

$$Cu_aThA_bO_x$$

wherein
A is an alkali metal,
a is from 0.1 to 4,
b is from 0 to 0.5, and
x is a number such that the valence requirements of the other elements for oxygen is satisfied,

is prepared by the steps of (I) precipitating at a temperature below 50°C the metals copper and thorium in the form of insoluble thermally decomposable compounds thereof using a precipitant comprising a carbonate and/or bicarbonate of an alkali metal and/or ammonium, the pH being adjusted to a final value in the range from 5.0 to 7.0, (II) recovering the precipitate obtained in step (I), and (III) thermally decomposing thermally decomposable compounds comprised in the precipitate recovered in step (II), the maximum temperature utilised in the thermal decomposition being in the range from 300 to 600°C. Also a catalyst composition of formula $Cu_a.ThO_2$ wherein a is from 0.1 to 4 and wherein at least 30% of the elemental copper particles are of a size less than or equal to 20 Angstroms.

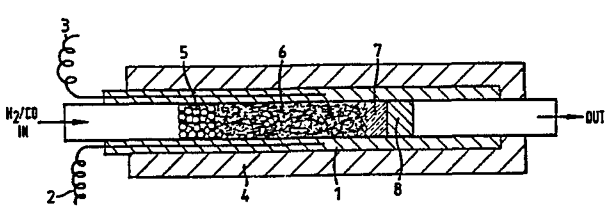


EP 0 171 178 A1

# CATALYST PROCESS FOR ITS PRODUCTION AND ITS US IN THE CONVERSION OF SYNGAS TO METHANOL

The present invention relates to a catalyst for use in a process for the production of methanol from synthesis gas, a process for the production of the catalyst and a process for the production of methanol utilising the catalyst.

Metal or metal oxide catalysts have long been known to catalyse processes for the production of oxygenated compounds, for example alcohols, from synthesis gas. Furthermore, copper and thorium have long been recognised as active components of such catalysts. See, for example, US Patents Nos. 1,707,331,; 2,061,470; 2,500,913; 1,741,307; 1,625,929 and 1,831,179.

US Patent No. 1,625,929 describes a methanol catalyst which comprises three main elements, i.e. (1) one or more difficultly reducible metal oxides such as zinc, magnesium, cadmium, chromium, vanadium, tungsten, uranium, zirconium, titanium, aluminium, manganese, molybdenum, thorium, cerium, etc., (2) one or more easily reducible metal oxides such as copper, silver, iron, nickel, cobalt, etc., (3) a metallic halide. The improvement in such catalysts is ascribed to the presence of the halide.

US Patent No. 1,707,331 describes a catalyst for the production of methanol from hydrogen and carbon monoxide which comprises copper and a rare earth metal fluoride. Suitable rare earth metals are defined as thorium, scandium, lanthanum, cerium, praseodymium, neodymium and samarium. Example 11, of this patent describes the preparation of a copper/thorium fluoride supported on asbestos catalyst by a hot precipitation method.

US Patent No. 1,741,307 describes a process of reducing carbon monoxide and carbon monoxide-containing gases which comprises causing the gas to react with hydrogen-containing gases in successive stages to form formaldehyde and methyl alcohol without isolating intermediate products formed, at least one of the first two stages taking place in the presence of mild reduction catalysts associated with oxidation catalysts, the latter acting as carrier for the former. Mild reduction catalysts include copper, manganese, cadmium, zinc, lead, tin, magnesium, silver, gold and platinum and oxidation catalysts include compounds of chromium, vanadium, manganese, titanium, molybdenum, tungsten, cerium, thorium, uranium, zirconium and the like. In Example 2 of this patent there is described a process producing methanol using a layered catalyst in which one component (2) is thorium oxide coated with copper and zinc produced by evaporating complex ammonium salts of copper and zinc nitrate on the thorium oxide fragments.

US Patent No. 1,831,179 describes a similar process to the stagewise process of US Patent No. 1,741,307. In Example I of this patent the catalyst used in the first stage reaction of carbon dioxide with hydrogen to produce carbon monoxide and water consists of 100 parts pure copper carbonate on 1000 parts thorium oxide fragments, the copper carbonate being stuck on with dextrin and reduced at 280°C in a stream of hydrogen.

US Patent No. 2,061,470 describes the production of methanol from synthesis gas using a catalyst consisting of a reduction product of a fused mixture of copper oxide and manganese oxide, or copper oxide and zinc oxide, in which there is incorporated one or more of the elements magnesium, aluminium, chromium, zirconium, vanadium, titanium, thorium, silicon and cerium. Example 7 of this patent describes a catalyst consisting of 78 parts of pure cupric oxide, 20 parts of pure manganese dioxide and 2 parts of pure thorium oxide.

US Patent No. 2,500,913 describes oxo-synthesis catalysts prepared by impregnating a gel comprising silica with salts of cobalt, thorium and copper.

Of the aforesaid catalysts, only that of US Patent No. 1,707,331 is prepared by a precipitation technique and that involves precipitation from a hot solution.

Finally, US Patent No. 4,298,354 describes a process for producing alcohol mixtures which comprises contacting a gaseous reactant containing carbon monoxide and hydrogen with an oxide complex catalyst described by the formula:

$$Cu_aThM_bA_cO_x$$

wherein

M is one or more of Ca,Mo,Rh,Mn,Pt,Ce,Cr,Zn,Al,Ti,La,V,U,Ru,Re and Pd;

A is an alkali metal; and

wherein

a is 0.5 to 2.5;

b is 0.01 to 1.0;

c is 0.05 to 0.9; and

x is a number such that the valence requirements of the other elements for oxygen is satisfied.

The catalyst of US Patent No. 4,298,354 is prepared by a procedure involving adding an alkali metal carbonate to an aqueous solution containing decomposable salts of thorium, copper and the "M" element, preferably maintained at an elevated temperature, e.g. 80 to 95°C, until the pH of the system increases to at least 7.5, preferably at least 8, most preferably about 9.3 to 10, digesting the precipitate whilst maintaining the temperature, acidifying to pH 6.8 to 7, separating and washing the precipitate, drying the precipitate and thereafter calcining the precipitate at a temperature sufficient to drive out the remaining water and decompose the decomposable ions remaining therein. In Example 1 and subsequent Examples the precipitation step is carried out at a temperature of 90-95°C.

The present invention provides a process for the production of a catalyst precursor for use after reductive activation in the conversion of synthesis gas to methanol, the precursor having the formula:

$$Cu_aThA_bO_x \qquad (I)$$

wherein A is an alkali metal,

a is from 0.1 to 4

b is from 0 to 0.5, and

x is a number such that the valence requirements of the other elements for oxygen is satisfied

which process comprises the steps of :

(I) precipitating at a temperature below 50°C the metals copper and thorium in the form of insoluble thermally decomposable compounds thereof using a precipitant comprising a carbonate and/or bicarbonate of an alkali metal and/or ammonium, the pH being adjusted to a final value in the range from 5.0 to 7.0,

(II) recovering the precipitate obtained in step (I), and

(III) thermally decomposing heat decomposable compounds comprised in the precipitate recovered in step (II), the maximum temperature utilised in the thermal decomposition being in the range from 300 to 600°C.

Preferably a in the formula (I) has a value in the range from 0.5 to 3, even more preferably from 0.7 to 2. Preferably b in the formula (I) has a value less than 0.15, even more preferably zero.

Other metals M may be incorporated in the catalyst precursor of formula (I), some with advantageous effects. Suitable metals M include Mo,Rh,Mn,Pt,Ce,Cr,Zn, Al,Ti,La,V,U,Ru,Re,Pd,Ag, Au and akaline earth metals, for example Ca. The metals M may be incorporated in amounts up to 20% w/w.

After reductive activation, the product of the process of the invention is an excellent methanol production catalyst. Very high methanol space time yields and methanol selectivities as high as 99% can be achieved. Moreover, the catalyst is resistant to decay and is very reproducible. By comparison, a catalyst produced by precipitation at more elevated temperatures, for example 90°C, but under otherwise identical conditions is very much less active for methanol production.

Step I of the aforesaid process may be accomplished in a variety of ways, some being better than others in terms of the

activity of the final catalyst. Thus, an embodiment (A) comprises bringing together in solution at a temperature below 50°C soluble salts of the metals copper and thorium and a precipitant comprising a carbonate and/or a bicarbonate of an alkali metal and/or ammonium, the pH being adjusted to a final value in the range from 5.0 to 7.0. Alternatively, an embodiment (B) comprises bringing together in a substantially copper-free solution a soluble thorium salt with a precipitant so as to precipitate a thorium compound and thereafter in the presence of the precipitated thorium compound bringing together a solution of a soluble copper salt with a precipitant so as to precipitate an insoluble thermally decomposable compound, the pH being adjusted to a final value in the range from 5.0 to 7.0, said precipitations being carried out at a temperature below 50°C and said precipitant comprising a carbonate and/or a bicarbonate of an alkali metal and/or ammonium. After precipitation of the thorium compound, the solution of the soluble copper salt may suitably be added to the precipitated thorium compound without any intervening treatment, such as for example filtration, prior to precipitating the copper compound. Alternatively, the precipitated thorium compound may be separated, washed and re-dispersed prior to precipitating the copper compound. Many variants on the aforesaid embodiments (A) and (B) are possible, for example instead of adding the precipitant to the salts, the salts may be added to the precipitant.

Whilst any soluble salt of copper and thorium may be employed, such as for example carboxylates, acetyl-acetonates, naphthenates, chlorides and the like, it is preferred to use the nitrates.

It is preferred to use aqueous solutions of the salts, though aqueous alcoholic solutions for example may be employed if desired.

Of the alkali metals sodium and potassium are suitable, principally for reasons of availability and cost. Examples of suitable precipitants are sodium carbonate, sodium bicarbonate, potassium carbonate, potassium bicarbonate, ammonium carbonate and ammonium bicarbonate. Using alkali metal salts as the precipitant, the resulting catalyst precursor composition will almost certainly

contain alkali metal cations, the amount of which may be reduced by washing or may be increased by further addition of alkali metal cations. However, we have found that catalysts free from alkali metal [b in the formula (I) equal to zero] are the most active and for this reason it is preferred to use either ammonium carbonate or ammonium bicarbonate, even more preferably ammonium bicarbonate, as the precipitant. Ammonium carbonate may suitably be used in a commercially available form, which comprises a mixture of ammonium bicarbonate and ammonium carbonate. The use of ammonium bicarbonate as the precipitant unexpectedly provides the most active of the catalysts embraced within the scope of the present invention.

The precipitation may suitably be effected at a temperature below 30°C, even more preferably at a temperature in the range from 0 to 25°C. Precipitation at room temperature will usually be found most convenient.

The amounts of the soluble copper and thorium salts and precipitant employed should be such as to satisfy the values of a and b in the formula (I).

In order to improve the homogeneity of the catalyst it is preferred to agitate the mixture during precipitation, suitably by mechanical stirring.

Addition of the precipitant causes the initially low pH to rise. Generally, it will be found that precipitation of the thorium compound is substantially complete at a pH of about 4. It is important in the precipitation of the copper component of the catalysts that the final value of the pH is in the range from 5.0 to 7.0, preferably from 5.5 to 6.5, even more preferably from 5.9 to 6.3. The steps taken prior to achieving the final pH may vary considerably. Thus, the precipitant may be added until a pH in the aforesaid range is achieved, whereupon the addition of a further precipitant may be discontinued, thereby arresting the rise in the pH. Alternatively, the rise in pH may be allowed to continue by further addition of precipitant until it reaches a value in the range from 9 to 10 (at which point it will generally be found that precipitation is complete) and the pH of the mixture thereafter

adjusted to a final value in the aforesaid range by addition of acid. In another alternative, the pH of the mixture may be maintained at a value in the aforesaid range by the addition of a suitable buffer.

It is believed that the precipitate resulting from the process of the invention involving precipitation from the metal nitrates at a temperature below 50°C and especially below 30°C contains a crystalline phase comprising copper in the form of the basic carbonate, whereas the copper in the crystalline phase of the precipitate obtained by precipitation at more elevated temperatures comprises the copper hydroxy nitrate.

It is further preferred to carry out step (I) in an atmosphere of carbon dioxide. Though we do not wish to be bound in any way by theory, it is believed that the presence of carbon dioxide may stabilise any carbonate phase and improve the dispersion of the metallic phases in the composition.

In step (II) of the process of the invention the precipitate obtained in step (I) is recovered. This may suitably be accomplished by filtration but other methods for separating solids from liquids, for example centrifugation, may be employed. After recovery, it is preferred to wash the precipitate, suitably with water, so as to remove unwanted residual soluble matter. Thereafter, the precipitate may be dried, suitably at an elevated temperature below 150°C, for example about 120°C.

In step (III) of the process according to the invention heat decomposable compounds comprised in the precipitate recovered in step (II) are thermally decomposed. The maximum temperature used during the thermal decomposition must not exceed a temperature in the range from 300 to 600°C, preferably from 400 to 500°C, even more preferably from 440 to 460°C. The rate at which the temperature is increased may be varied over a wide range. It is preferred to maintain the precipitate at the maximum temperature for an extended period, for example from 1 to 200 hours. It is preferred to conduct the decomposition in a non-reducing atmosphere, suitably in a stream of either an inert gas, for example nitrogen, or an

oxygen-containing gas, for example, air.

After decomposition the product comprises a metal oxide composition convertible by reductive activation into a catalyst for the conversion of synthesis gas to methanol.

Catalyst precursor compositions obtained using ammonium bicarbonate as the precipitant (compositions of formula (I) in which b = 0) may be reductively activated in a reducing gas comprising carbon monoxide, for example in carbon monoxide/hydrogen-containing mixtures. This affords the advantage that precursor compositions so-derived can be reductively activated within a methanol production reactor using the same or different reaction conditions to those employed in the methanol production process.

For catalyst precursor compositions other than those referred to hereinabove, ie b in formula (I) greater than zero, reductive activation is preferably effected, at least initially, with reducing gas substantially free from carbon monoxide, for example hydrogen preferably diluted with inert gas. A very suitable gas mixture is 5% hydrogen in nitrogen. Following initial reduction with a reducing gas other than carbon monoxide, subsequent reduction may be effected in carbon monoxide-containing gases, for example carbon monoxide/hydrogen mixtures. Suitably reductive activation in this manner may be carried out as a discrete step prior to use as a catalyst for methanol production, either prior to loading into a methanol production reactor or within a methanol production reactor.

The pressure employed during the reductive activation step may suitably be in the range from 1 to 100 bar, the temperature may suitably be in the range from 170 to 250°C and periods of 24 hours or longer may be employed.

We have found that a characteristic of active catalysts is a relatively high proportion of elemental copper particles of a size less than or equal to 20 Angstroms.

Accordingly, in another aspect the present invention comprises a catalyst composition for use in the conversion of synthesis gas to methanol which composition comprises elemental copper and an oxide of thorium in the atomic ratio:

$Cu_aThO_x$ (II)

wherein x is a number such that the valence requirements of Th for oxygen is satisfied, and

a is in the range from 0.1 to 4,

and wherein at least 30% of the elemental copper particles are of a size less than or equal to 20 Angstroms.

The activity of the catalyst is related to the absolute amount of elemental copper particles of size less than or equal to 20 Angstroms and in consequence for any particular composition as high a proportion as possible of elemental copper particles of size less than or equal to 20 Angstroms is desirable.

Preferably at least 50%, even more preferably at least 90% of the elemental copper particles are of size less than or equal to 20 Angstroms.

Preferably a in the formula (II) has a value in the range from 0.5 to 3, even more preferably from 0.7 to 2.

According to another aspect of the present invention there is provided a process for the production of methanol from synthesis gas which process comprises contacting synthesis gas with a catalyst as hereinbefore described at a temperature in the range from 80 to 300°C and a pressure of from 0 to 100 bars.

As is well known in the art synthesis gas principally comprises carbon monoxide and hydrogen and possibly also minor amounts of carbon dioxide, nitrogen and other inert gases depending upon its origin and degree of purity. Methods for preparing synthesis gas are established in the art and usually involve the partial oxidation of a carbonaceous substance, e.g. coal. Alternatively, synthesis gas may be prepared, for example by the catalytic steam reforming of methane. For the purpose of the present invention the carbon monoxide to hydrogen ratio may suitably be in the range from 2:1 to 1:8, preferably from 1:1 to 1:3, even more preferably 1:2. Whilst the ratio of the carbon monoxide to hydrogen in the synthesis gas produced by the aforesaid processes may differ from these ranges, it may be altered appropriately by the addition of either carbon monoxide or hydrogen, or may be adjusted by the so-called shift

reaction well known to those skilled in the art. It may be preferred to remove carbon dioxide from the synthesis gas, suitably by scrubbing, prior to contact of the synthesis gas with the catalyst.

The synthesis gas is contacted with the catalyst at a temperature in the range from 80 to 300°C. Operation of the process at temperatures above 300°C tends to produce increasing amounts of higher alochols in addition to gaseous products, thereby decreasing the selectivity to methanol. It is preferred to operate at a temperature in the range from 180 to 250°C for the highest selectivities to methanol. Preferably the operating pressure is in the range from 20 to 100 bars. The Gas Hourly Space Velocity (GHSV) may be varied over a wide range, for example in the range from 1000 to 350,000 $hour^{-1}$.

The process may suitably be operated either in fluid-bed mode or fixed bed mode, and either batchwise or continuously, preferably continuously.

The catalyst may additionally contain materials, for example alumina or chromia, conventionally added for the purpose of dilution, controlling pellet density and providing other mechanical requirements, such as for example inhibition of shrinkage and abrasion resistance. These materials may be added at any convenient stage of catalyst preparation.

The invention will now be further illustrated by reference to the following Examples.

For the purpose of Examples 21 to 23, and 31 to 38, and Comparison Tests 4, 6 and 7, the tubular reactor illustrated in Figure 1 was employed. With reference to Figure 1, 1 is a stainless steel tube of 8mm internal diameter, there being supported around the reactor in slotted rings a recorder thermocouple 2 and a controller thermocouple 3, which thermocouples in conjunction with a CRL controller and Honeywell-Brown recorder (neither of which are shown) are used to control and monitor the temperature of the carbolite model CJT1/1000 split furnace 4. Synthesis gas entering the tubular reactor first encounters silica spheres 5 which act as a

preheater, followed by the catalyst 6, a ceramic wool plug 7 and finally stainless steel gauze 8 before exiting from the reactor.

The reactor fits into the unit layout in the manner shown in Figure 2. With reference to Figure 2, the principal features are a Brooks mass flow controller 9, a hydrogen by-pass 10, the reactor assembly of Figure 1, knock out pots 11 and 12, a three-way valve 13 and a pressure controller 14.

For the purpose of Examples 24 to 30, 39 to 44 and Comparison Test 5, a different reactor and layout, as shown in Figures 3 and 4, was employed. Figure 3 shows the reactor employed and Figure 4 shows the layout including the reactor of Figure 3.

With reference to Figure 3, 15 is a stainless steel tube of 7mm internal diameter, 16 is a stainless steel thermowell of 2.5mm internal diameter with a recorder thermocouple 17 positioned down its centre. A controller thermocouple 18 is positioned in a slotted ring parallel to the reactor tube. These thermocouples in conjunction with a eurotherm controller and a CRL recorder (neither of which are shown) are used to control and monitor the temperature of the Stanton Redcroft low mass horizontal furnace 19. Synthesis gas entering the tubular reactor first encounters a ceramic wool plug 20, followed by the catalyst 21 and finally another ceramic wool plug 22 before exiting from the reactor.

The reactor assembly of Figure 3 fits into the unit layout in the manner shown in Figure 4. With reference to Figure 4, gases are introduced to the plant from cylinders through a pressure regulator 23, and the flow rates are controlled by post-reactor needle valves 24 and 25. Flow is measured by bubble meters on both the reactor vent and the G.C. vent. A trap-pot is available for product sampling when required.

Preparation of Catalyst Precursors

Example 1 - Catalyst Precursor A (precipitation at low temperature)

Copper nitrate, $Cu(NO_3)_2 3H_2O$ (120g) and thorium nitrate, $Th(NO_3)_4 4H_2O$(180g) were dissolved in distilled water (3000 ml). Potassium carbonate (207.3g, anhydrous) was dissolved in distilled water (1500ml) and the resulting solution was added dropwise with

stirring to the copper-thorium solution at room temperature (ca 20°C). The final pH of the mixture was 6 using 1150 $cm^3$ of carbonate solution. The suspension was filtered and the filter-cake washed by resuspension in distilled water (3 x 3000 ml). The solid was then dried at 120°C and calcined in air at 450°C for 6 hours, the temperature being raised from 20°C to 450°C over 60 minutes. Analysis of the catalyst precursor showed copper 19%, thorium 44% and potassium 1.37% b/w.

Comparison Test 1 - Catalyst Precursor B (precipitation at 80 to 90°C)

The preparation was the same as that described for Catalyst Precursor A in Example 1 except that the copper nitrate/thorium nitrate solution and the potassium carbonate solution were heated to 80-90°C before mixing. After calcination, analysis of the catayst precursor showed copper 22%, thorium 44% and potassium 1.18% by weight.

This is not an example according to the present invention because the precipitation was carried out at 80-90°C.

Example 2 - Catalyst Precursor C (variation in order of addition of precipitant)

Copper nitrate trihydrate (20g) was dissolved in distilled water (250 ml) and 1 molar solution of potassium carbonate was added slowly with stirring until the pH of the mixture was 6.0. Thorium nitrate hexahydrate (30g) was dissolved in distilled water (250ml) and the resulting solution added to the above copper suspension. A 1 molar solution of potassium carbonate was then added slowly with stirring to bring the pH to 6.0. The mixture was filtered and the filter-cake washed by resuspension in distilled water (3 x 500ml) and finally dried at 120°C for 16 hours.

The solid was thermally decomposed at 450°C in air, the temperature being raised rapidly to 50°C and then programmed at 30°C/h to 450°C and maintained at that temperature for 6 hours.

Example 3 - Catalyst Precursor D (precipitation with potassium bicarbonate)

The procedure of Example 1 (Catalysts Precursor A) was repeated

except that ammonium bicarbonate was employed in place of potassium carbonate.

Example 5 - Catalyst Precursor F (precipitation at pH5)

The procedure of Example 1 (Catalyst Precursor A) was repeated except that aqueous potassium carbonate was added only until pH5 was reached, the preparation was scaled down and the thermal decomposition of the dried solid was accomplished at 450°C, the temperature being raised rapidly to 50°C and then programmed at 30°C/h to 450°C, at which temperature it was held for 6h.

Example 6 - Catalyst Precursor G (precipitation at pH6)

The procedure of Example 5 (Catalyst Precursor F) was repeated except that aqueous potassium carbonate was added until pH6 was reached.

Example 7 - Catalyst Precursor H (precipitation at pH7)

The procedure of Example 5 (Catalyst Precursor F) was repeated except that aqueous potassium carbonate was added until pH7 was reached.

Comparison Test 2 - Catalyst Precursor I (precipitation at pH8)

The procedure of Example 5 (Catalyst Precursor F) was repeated except that aqueous potassium carbonate was added until pH8 was reached.

This is not an example according to the present invention because the final pH was not in the range 5 to 7.

Example 8 - Catalyst Precursor I′(Sequential precipitation of embodiment B)

Copper nitrate, $Cu(NO_3)_2.3H_2O$, (24.0g, 99.3 mmole) was dissolved in deionised water (20ml) to form Solution X. Thorium nitrate, $Th(NO_3)_4.6H_2O$, (36.0g, 61.2 mmoles) was dissolved in deionised water (600 ml) to form Solution Y. Ammonium bicarbonate, $NH_4HCO_3$, (47.6g, 598.0 mmoles) was dissolved in deionised water (300 ml) and this solution was added dropwise at a rate of 5 ml/minute with stirring to Solution Y at room temperature until the pH reached a value of 4, at which point addition was stopped. Stirring was continued for a further 15 minutes and the suspension filtered. The filter-cake was washed by immersion in deionised

water (300 ml), stirred for 15 minutes and filtered. The washed filter-cake was redispersed in deionised water (680 ml) and Solution X then added to the dispersion. Ammonium bicarbonate addition was recommenced in the manner described above until the pH reached a value of 6. Stirring was continued for a further 15 minutes and the suspension filtered. The filter-cake was washed three times in the manner hereinbefore described. The product was then dried at 110°C for 17 hours and broken down to form granules passing 16 mesh but not 30 mesh (BSS).

The product was decomposed by a controlled programmed heat-treatment in a flow of nitrogen. The temperature was raised from ambient to 450°C at 120°C per hour and then maintained at 450°C for 6 hours before cooling.

<u>Example 9 - Catalyst Precursor J</u>

Example 8 was repeated except that the composition was changed in the respect that the amount of copper nitrate was reduced to 14.8g (61.3 mmole).

<u>Example 10 - Catalyst Precursor K</u>

Copper nitrate, $Cu(NO_3)_2.3H_2O$, (14.8g, 61.3 mmole) was dissolved in deionised water (20 ml) to form Solution X. Thorium nitrate, $Th(NO_3)_4.6H_2O$, (36.0g, 61.2 mmoles) was dissolved in deionised water (600 ml) to form Solution Y. Ammonium bicarbonate,$NH_4HCO_3$, (47.6g, 598.0 mmoles) was dissolved in deionised water (300 ml) and this solution was added dropwise at a rate of 5ml/minute with stirring to Solution Y at room temperature until the pH reached a value of 4, at which point ammonium bicarbonate addition was stopped. Stirring was continued for 15 minutes and thereafter Solution X was added to the suspension. To this mixture was added the ammonium bicarbonate solution in the same manner as before until the pH reached a value of 6. Stirring was continued for a further 15 minutes and the suspension was then filtered.

The product was then decomposed in the manner described in Example 8 for Catalyst Precursor I′.

The Example differs from Example 9 in that the precipitated

thorium compound was not separated, washed and redispersed before addition of the copper salt solution and precipitation of copper therefrom.

Comparison Test 3.- Catalyst Precursor L

Thorium nitrate, $Th(NO_3)_4.6H_2O$, (36.0g, 61.2 mmoles) was dissolved in deionised water (600 ml) to form Solution Y. Ammonium bicarbonate, $NH_4HCO_3$, (47.6g, 598.0 mmoles) was dissolved in deionised water (300 ml) and added dropwise at a rate of 5ml/minute with stirring to Solution Y at room temperature until the pH reached a value of 4. Stirring was continued thereafter for a period of 15 minutes and the suspension filtered. The filter-cake was washed by immersion in deionised water (300 ml), stirred for 15 minutes and filtered. This washing operation was carried out 3 times in total. The product was then dried at 110°C for 17 hours.

The product obtained above (15.0g) was ground to a fine powder and added to deionised water (10ml). Copper nitrate, $Cu(NO_3)_2.3H_2O$, (10.3g, 42.7 mmoles) was dissolved with stirring in the suspension of the thorium compound and the suspension evaporated to dryness in 6 hours on a rotary evaporator. The final product was then dried at 110°C for 7 hours.

After drying the product obtained was decomposed in the manner described in Example 8 for Catalyst Precursor I'.

This is not an example according to the present invention because the copper was not precipitated.

Example 11 - Catalyst Precursor M

Copper nitrate, $Cu(NO_3)_2.3H_2O$, (24.0g, 99.3 mmoles) and thorium nitrate, $Th(NO_3)_4.6H_2O$, (36.0g, 61.2 mmoles) were dissolved in deionised water (600 ml) to form Solution W. Ammonium bicarbonate ($NH_4HCO_3$, 47.6g, 598 mmoles) was dissolved in deionised water (300 ml) and this solution was added dropwise at a rate of 5ml/minute with stirring to Solution W at room temperature until the pH reached a value of 6. Stirring was continued for a further 15 minutes and the suspension filtered. The filter-cake was washed by immersion in deionised water (300 ml), stirred for 15 minutes and filtered. The washing operation was carried out a total of three times. The

product was then dried at 110°C for 17 hours.

Finally, the dry product was decomposed in the manner described in Example 8 for Catalyst Precursor I′.

The thorium was not precipitated in the substantial absence of copper. In this respect it differs from Example 8.

Example 12 - Catalyst Precursor N

Example 11 was repeated except that the composition was changed in the respect that the amount of copper nitrate was reduced to 14.8g (61.3 mmoles).

The thorium was not precipitated in the substantial absence of copper. In this respect it differs from Example 9.

Example 13 - Catalyst Precursor O (precipitation with ammonium hydrogen carbonate)

Copper nitrate, $Cu(NO_3)_2.3H_2O$, (14.8g, 61.3 mmol) and thorium nitrate, $Th(NO_3)_4.6H_2O$, (36.0g, 61.2 mmol) were dissolved in deionised water (600 ml) to form Solution X. Ammonium hydrogen carbonate, $NH_4HCO_3$, (47.6g, 600 mmol) was dissolved in deionised water (Solution Y). This solution, i.e. Solution Y, was added dropwise at room temperature to the vigorosly stirred Solution X until a pH of 6.0 was reached. After continuing the stirring for 15 minutes, the suspension was filtered. The filter cake was washed by immersing in deionised water (600 mols). This washing operation was carried out three times in all. The final solid cake was then dried at 110°C for 17 hours. The product was then broken down to pass 16 but not 30 mesh (BSS).

The new product was then decomposed by a programmed heat treatment in a flow of nitrogen. The temperature was raised from ambient to 450°C at 120°C per hour and then maintained at 450°C for 6 hours.

Example 14 - Catalyst Precursor P

This was prepared in an identical manner to Catalyst Precursor O in Example 13 using as Solution X a mixture of the nitrates, $Cu(NO_3)_2.3H_2O$ (24.0g, 99.3 mmol), $Ce(NO_3)_3.6H_2O$ (1.3g, 3.1 mmol) and $Th(NO_3)_4.6H_2O$ (36.0g, 61.2 mmol).

Example 15 - Catalyst Precursor Q (thermal decomposition at 300°C)

The procedure of Example 1 (Catalyst Precursor A) was repeated excepted that, after drying, a portion of the dry solid was thermally decomposed in air by raising the temperature rapidly to 50°C and then in a programmed manner at 30°C/h to 300°C and held at that temperature for 6h.

Example 16 - Catalyst Precursor R (thermal decomposition at 400°C)

A further portion of the dry solid obtained in Example 15 was thermaly decomposed in the manner described in Example 15 except that the final temperature was 400°C instead of 300°C.

Example 17 - Catalyst Precursor S (thermal decompostion at 450°C)

A further portion of the dry solid obtained in Example 15 was thermally decomposed in the manner described in Example 15 except that the final temperature was 450°C instead of 300°C.

Example 18 - Catalyst Precursor T (thermal decomposition at 500°C)

A final portion of the dry solid obtained in Example 15 was thermally decomposed in the manner described in Example 15 except that the final temperature was 500°C instead of 300°C.

Example 19 - Catalyst Precursor U

The procedure of Example 1 (Catalyst Precursor A) was employed except the thermal decomposition of the dried solid was accomplished at 450°C, the temperature having been raised rapidly to 50°C and then in a programmed manner at 30°C/h to 450°C at which temperature it was held for 6h.

Example 20 - Catalyst Precursor V

Copper nitrate, $Cu(NO_3)_2.3H_2O$, (48.0g, 198.6 mmole) was dissolved in deionised water (40 ml) to form a solution designated X. Thorium nitrate, $Th(NO_3)_4.6H_2O$, (72.0g, 122.4 mmole), was dissolved in deionised water (120 ml) to form a solution designated Y. This solution was kept saturated with carbon dioxide gas throughout the preparation. Ammonium bicarbonate, $NH_4HCO_3$, (95.2g, 1196 mmole) was dissolved in deionised water (600 ml) and this solution was added dropwise at a rate of 10 ml/minute with stirring to solution Y at room temperature until the pH reached a value of 4, at which point the addition was stopped. Stirring was continued for a further 15 minutes and thereafter solution X was added to the

suspension. To this solution was added further ammonium bicarbonate solution in the same manner as before until the pH reached a value of 6. Stirring was continued for a further 15 minutes and the suspension was then filtered. The filter-cake was washed three times in the manner hereinbefore described. The product was then decomposed in the manner described in Example 8.

Conversion of catalyst precursors into catalysts and testing of catalysts so-obtained

(A) Effect of variations in step (I) of the process of the invention

Example 21 - Catalyst A

With reference to Figures 1 and 2, Catalyst Precursor A was packed into the reactor illustrated in Figure 1 and reductively activated by passage of 5% hydrogen in nitrogen at 170°C for 16h, the temperature having been raised to 170°C over a period of 1h.

Thereafter, the flow of $H_2/N_2$ was cut off and carbon monoxide and hydrogen were fed from cylinders in a ratio of $CO:H_2$ = 1:2 (molar) to the reactor via pressure control valves and Brooks' thermal mass-flow controllers 9. The inlet pressure was indicated by a gauge and the plant was protected against overpressure by a bursting disc. The system was pressurised to 50 bars over 30 minutes. At that pressure the flows were settled out at a GHSV of $7000h^{-1}$.

Gaseous products passing from the reactor were fed through heated lines and a selector valve 13 to one of two traps 11 and 12 arranged in parallel. The two traps were cooled in solid carbon dioxide (cardice)/acetone to -5 to -10°C, one trap being used for the running-in period and the other for the balanced run. Gases leaving these traps were fed first to a pressure controller 14 in which the pressure was dropped to atmospheric and then to a gasmeter, a sampling point and finally to vent.

After one hour, when steady running conditions had been achieved, the selector valve 13 was switched and a run of one hour duration was carried out collecting the liquid product in the previously unused cold trap. The vent gas was measured by a

gasmeter and a vent gas sample was taken during the run. Liquid and gaseous products were analysed by gas chromatography. The result in terms of methanol productivity is shown in Table 1.

Comparison Test 4 - Catalyst B

The procedure of Example 21 was repeated except that Catalyst Precursor B was used in place of Catalyst Precursor A.

Table 1

| Example | Catalyst | Methanol productivity g mol/h/l of catalyst |
|---|---|---|
| 21 | A | 43 |
| Comp Test 4 | B | 25 |

The results shown in Table 1 clearly demonstrate that the catalyst derived from the precursor precipitated at room temperature is more active than a catalyst derived from a catalyst precursor precipitated at 80 to 90°C.

Example 22

The procedure of Example 21 was repeated except that Catalyst Precursor A was replaced by Catalyst Precursor C. The result in terms of methanol productivity together with that for Catalyst A is given in Table 2.

Table 2

| Example | Catalyst | Methanol productivity g mol/h/l of catalyst |
|---|---|---|
| 21 | A | 43 |
| 22 | C | 46 |

Example 23

The procedure of Example 21 was repeated except that Catalyst Precursor A was replaced by Catalyst Precursor D. The result in terms of methanol productivity together with that for Catalyst A is given in Table 3.

Table 3

| Example | Catalyst | Precipitant | Methanol productivity g mol/h/l of catalyst |
|---|---|---|---|
| 21 | A | potassium carbonate | 43 |
| 23 | D | potassium bicarbonate | 53 |

Example 24

With reference to Figure 4, Catalyst Precursor E (1 ml) was diluted to 4 ml using inert carborundum and placed in the reactor giving a 5cm length bed, the diluent being used to control the heat of reaction.

The catalyst was reductively activated by passage of 5% hydrogen in nitrogen at 170°C for 16h, the temperature having been raised to 170°C over a period of 1 hour.

After reductive activation, the flow of $H_2/N_2$ was cut off and a mixture of carbon monoxide and hydrogen in a molar ratio of $CO:H_2$ of 1:2 was fed at a GHSV of 100,000 $h^{-1}$ to the reactor maintained at a temperature of 200°C and an absolute pressure of 50 bars.

Effluent gas was carried in heated lines (200°C) to a Pye Unicam Gas Chromatographic Detector, with a 2ml sampling loop. Using helium as a carrier gas, products were separated with a 2 metre column of Porapak Q at 112°C. This gave good separation of CO, $CO_2$, $H_2O$ and MeOH. Calibration was carried out by the injection of 0.5 microlitre aliquots of liquid methanol. A Pye Unicam D4810 integrator was used to measure the peak areas. The typical methanol retention time on this column was 250s. The result in terms of methanol productivity is given in Table 4.

Example 25

The procedure of Example 24 was repeated except that instead of Catalyst Precursor E there was used a catalyst precursor prepared in identical manner to Catalyst Precursor A as described in Example 1, i.e. using potassium carbonate as the precipitant. The result in terms of methanol productivity is given in Table 4.

Table 4

| Example | Catalyst | Precipitant employed | Methanol productivity g moles/h/l of catalyst |
|---|---|---|---|
| 24 | E | ammonium bicarbonate | 87 |
| 25 | A | potassium carbonate | 15 |

The results in Table 4 demonstrate that more active catalysts are produced using ammonium bicarbonate rather than potassium carbonate in the preparative process.

Examples 26 to 30

With reference to Figures 3 and 4, a sample of the catalyst precursor I′ was packed into the reactor illustrated in Figure 3. Synthesis gas was introduced to the reactor at ambient temperature and thereafter the temperature was raised to 180°C over 1 hour. The catalyst was allowed to stabilise for 24 hours before measuring the catalyst activity. In Examples 27 to 30 the procedure was repeated using catalyst precursors J and K and M and N respectively.

The prevailing conditions and the results obtained in terms of %MeOH and %$CH_4$ in the product stream are given in the following Table 5.

Comparison Test 5

The procedure of Examples 26 to 30 was repeated using catalyst precursor L.

Table 5

| Example | Catalyst precursor | Pressure (Atmos. Absolute) | GHSV ($h^{-1}$) | Temp. (°C) | MeOH** | $CH_4$** |
|---|---|---|---|---|---|---|
| 26 | Ex.8 (I′) | 9.3 | 3600 | 180 | 3.16 | * |
| 27 | Ex.9 (J) | 9.3 | 3600 | 181 | 2.11 | * |
| 28 | Ex.10 (K) | 9.3 | 3600 | 180 | 3.14 | 0.02 |
| Comp. Test 5 | Comp. Test 3 (L) | 9.3 | 3600 | 180 | None detected | - |
| 29 | Ex.11 (M) | 9.3 | 3600 | 181 | 2.50 | 0.02 |
| 30 | Ex.12 (N) | 9.3 | 3600 | 180 | 2.26 | 0.03 |

* not found
** in terms of % by volume in vent gas

Example 31

The procedure of Example 21 was repeated except that Catalyst Precursor A was replaced by Catalyst Precursor F. The result in terms of methanol productivity is shown in Table 6.

Example 32

The procedure of Example 31 was repeated except that Catalyst Precursor F was replaced by Catalyst Precursor G.

Example 33

The procedure of Example 31 was repeated except that Catalyst Precursor F was replaced by Catalyst Precursor H.

Comparison Test 6

The procedure of Example 31 was repeated except that Catalyst Precursor F was replaced by Catalyst Precursor I.

Table 6

| Example | Catalyst | pH of precipitation | Methanol productivity g mol/h/1 of catalyst |
|---|---|---|---|
| 31 | F | 5 | 24 |
| 32 | G | 6 | 43 |
| 33 | H | 7 | 27 |
| Comp Test 6 | I | 8 | 21 |

It can be seen from Table 6 that the activity of the catalyst depends upon the final pH at which the catalyst precursor was precipitated.

(B) Effect of variations in step (III) of the process of the invention

Example 34

The procedure of Example 21 was repeated except that Catalyst Precursor A was replaced by Catalyst Precursor Q. The result in terms of methanol productivity is given in Table 7.

Example 35

The procedure of Example 34 was repeated except that Catalyst Precursor Q was replaced by Catalyst Precursor R.

Example 36

The procedure of Example 34 was repeated except that Catalyst Precursor Q was replaced by Catalyst Precursor S.

Example 37

The procedure of Example 34 was repeated except that Catalyst Precursor Q was replaced by Catalyst Precursor T.

Table 7

| Example | Catalyst | Thermal decomposition temperature (°C) | Methanol productivity g mol/h/1 of catalyst |
|---|---|---|---|
| 34 | Q | 300 | 21 |
| 35 | R | 400 | 38 |
| 36 | S | 450 | 43 |
| 37 | T | 500 | 33 |

(C) Effect of reductive activation step

Example 38

The procedure of Example 21 was repeated except that Catalyst Precursor A was replaced by a portion of Catalyst Precursor U. The result in terms of methanol productivity is given in Table 8.

Comparison Test 7

The procedure of Example 21 was repeated except that Catalyst Precursor A was replaced by another portion of Catalyst Precursor U and the reductive activation treatment was omitted. The result in terms of methanol productivity is given in Table 8.

Table 8

| Example | Catalyst | Reductive activation | Methanol Productivity g mol/h/1 of catalyst |
|---|---|---|---|
| 38 | U | Yes | 43 |
| Comp Test 7 | U | No | 6 |

The results in Table 8 demonstrate that reductive activation is necessary for improving catalyst activity.

Example 39

With reference to Figures 3 and 4, a sample of Catalyst Precursor O was packed in the reactor illustrated in Figure 3 and activated by pretreatment with 5% $H_2$ in He at 170°C and 7 bars (Absolute) using a GHSV of 10,000 for 17 hours.

Following the pretreatment, the $H_2$/He gas flow was stopped, synthesis gas was introduced and the conditions rapidly adjusted to those required for catalyst testing as shown in the following Table 9. The product gas stream was sampled after 24 hours.

Example 40

Example 39 was repeated except that Catalyst Precursor P was used instead of Catalyst Precursor O.

Example 41

With reference to Figures 3 and 4, a sample of Catalyst Precursor O was packed into the reactor illustrated in Figure 3. The catalyst was purged with synthesis gas ($H_2$:CO ratio of 2:1) at the flow rate and pressure shown in Table 9. The temperature was raised from ambient to 180°C over a 1 hour period. The product gas stream was analysed after 24 hours.

Example 42

Example 41 was repeated except that Catalyst Precursor P was used instead of Catalyst Precursor O.

The results of the product gas stream analyses for Examples 39 to 42 are given in the following Table 9.

TABLE 9

| Catalyst Precursor | Example | Pressure (Bars A) | GHSV ($h^{-1}$) | Temperature (°C) | MeOH* |
|---|---|---|---|---|---|
| O | Example 39 | 9.3 | 3,600 | 180 | 2.17 |
| | Example 41 | 9.3 | 3,600 | 180 | 2.26 |
| P | Example 40 | 9.3 | 3,600 | 180 | 1.91 |
| | Example 42 | 9.3 | 3,600 | 180 | 2.02 |

* in terms of % by volume in vent gas

The results reported in Table 9 demonstrate that the activity of the catalysts obtained by reduction with synthesis gas under methanol production conditions is at least as high as the activity of the catalysts obtained by prior reduction with $H_2$/He mixtures under different conditions.

Example 43

Example 26 was repeated except that Catalyst Precursor I′ was replaced by Catalyst Precursor V.

Example 44

Example 43 was repeated except that Catalyst Precursor V was replaced by a Catalyst Precursor produced in identical manner to Catalyst Precursor V except that carbon dioxide was omitted. The resultant composition is designated Catalyst Precursor W.

The prevailing conditions and the results obtained in terms of %MeOH and %$CH_4$ in the product stream are given in the following Table 10.

Table 10

| Example | Catalyst | Pressure (Atmos. Absolute) | GHSV ($h^{-1}$) | Temp (°C) | MeOH** | $CH_4$** |
|---|---|---|---|---|---|---|
| 43 | V | 9.3 | 3600 | 180 | 3.18 | 0.04 |
| 44 | W | 9.3 | 3600 | 180 | 2.92 | 0.04 |

** in terms of % by volume in vent gas

Catalyst Characterisation

X-ray Diffraction (XRD)

A range of catalysts corresponding to the formula:

$$Cu_aThO_x$$

wherein a has a value in the range from 0.1 to 4 and x is a number such that the valence requirements of Th for oxygen is satisfied

were made by the aforedescribed low temperature (below 50°C) techniques. The proportion of elemental copper particles of size less than or equal to 20 Angstroms was determined indirectly by standard quantitative X-ray Diffraction analysis using an X-ray powder diffractometer employing Cu K alpha radiation. In essence

the amount of elemental copper particles of size greater than 20 Angstroms was measured and the value obtained was subtracted from the known total amount of copper to arrive at a figure for the amount of elemental copper particles of size less than or equal to 20 Angstroms.

The results of the XRD determinations on the catalysts and the activity of the catalysts for the conversion of synthesis gas to methanol are given in Table 11, the results being plotted in the form of a graph in Figure 5.

In the Table and the Figure:

R represents the molar ratio of the total copper and total thoria in the catalyst,

%Cu represents the percentage of elemental copper with a particle size less than or equal to 20 Angstroms,

A represents the percentage of methanol synthesised by the sample under standard test conditions per gram of thoria in the sample, and

G represents the number of grams of copper with a particle size less than or equal to 20 Angstroms associated with a gram of thoria.

Table 11

| R | %Cu | A | G |
|---|---|---|---|
| 0.10 | 100 | 0.12 | 0.024 |
| 0.25 | 100 | 0.28 | 0.060 |
| 1.60 | 33 | 0.55 | 0.125 |
| 1.60 | 42 | 0.98 | 0.162 |
| 1.40 | 77 | 1.35 | 0.259 |
| 1.00 | 100 | 1.48 | 0.241 |
| 1.60 | 74 | 1.67 | 0.285 |

It can be seen from Table 11 and Figure 5 that there is a direct relationship between the methanol production activity of a catalyst (A) according to the invention and the number of grams of copper with a particle size less than or equal to 20 Angstroms associated with a gram of thoria (G). Over the compositional range studied A is directly proportional to G within the limits of experimental error.

Claims:

1 A process for the production of a catalyst precursor for use after reductive activation in the conversion of synthesis gas to methanol, the precursor having the formula:

$$Cu_aThA_bO_x \qquad (I)$$

wherein A is an alkali metal

a is from 0.1 to 4

b is from 0 to 0.5, and

x is a number such that the valence requirements of the other elements for oxygen is satisfied.

which process comprises the steps of:

(I) precipitating at a temperature below 50°C the metals copper and thorium in the form of insoluble thermally decomposable compounds thereof using a precipitant comprising a carbonate and/or bicarbonate of an alkali metal and/or ammonium, the pH being adjusted to a final value in the range from 5.0 to 7.0,

(II) recovering the precipitate obtained in step (I), and

(III) thermally decomposing heat decomposable compounds comprised in the precipitate recovered in step (II), the maximum temperature utilised in the thermal decomposition being in the range from 300 to 600°C.

2 A process according to claim 1 wherein a in the formula (I) has a value in the range from 0.5 to 3 and b is zero.

3 A process according to either claim 1 or claim 2 wherein step (I) comprises bringing together in solution at a temperature below 50°C soluble salts of the metals copper and thorium and a precipitant comprising a carbonate and/or a bicarbonate of an alkali metal

and/or ammonium, the pH being adjusted to a final value in the range from 5.0 to 7.0.

**4** A process according to either claim 1 or claim 2 wherein step (I) comprises bringing together in a substantially copper-free solution a soluble thorium salt with a precipitant so as to precipitate an insoluble thermally decomposable thorium compound and thereafter in the presence of the precipitated thorium compound bringing together a solution of a soluble copper salt with a precipitant so as to precipitate an insoluble thermally decomposable copper compound, the pH being adjusted to a final value in the range from 5.0 to 7.0, said precipitations being carried out at a temperature below 50°C and said precipitant comprising a carbonate and/or a bicarbonate of an alkali metal and/or ammonium.

**5** A process according to claim 4 wherein after precipitation of the thorium compound, the solution of the soluble copper salt is added to the precipitated thorium compound without any intervening treatment prior to precipitating the copper compound.

**6** A process according to claim 4 wherein the precipitated thorium compound is separated, washed and re-dispersed prior to precipitating the copper compound.

**7** A process according to any one of the preceding claims wherein the copper and thorium are in the form of aqueous solutions of their nitrate salts.

**8** A process according to any one of the preceding claims wherein the precipitant is either ammonium carbonate or ammonium bicarbonate.

**9** A process according to any one of the preceding claims wherein precipitation is effected at a temperature below 30°C.

**10** A process according to claim 9 wherein the temperature is in the range from 0 to 25°C.

**11** A process according to any one of the preceding claims wherein in step (I) the final value of the pH is in the range from 5.5 to 6.5.

**12** A process according to any one of the preceding claims wherein precipitation is carried out in an atmosphere of carbon dioxide.

**13** A process according to any one of the preceding claims wherein the precipitate recovered in step (II) is washed and dried at a temperature below 150°C.

**14** A process according to any one of the preceding claims wherein in step (III) the maximum temperature utilised is in the range from 400 to 500°C.

**15** A process according to any one of the preceding claims wherein in step (III) the thermal decomposition is conducted in a stream of either nitrogen or air.

**16** A process according to any one of the preceding claims wherein b in the formula (I) is zero and the composition is reductively activated in a reducing gas comprising carbon monoxide.

**17** A process according to claim 16 wherein the composition is reductively activated in a carbon monoxide/hydrogen-containing mixture.

**18** A process according to any one of claims 1 to 15 wherein b in the formula (I) is greater than zero and the composition is reductively activated, at least initially, with reducing gases substantially free from carbon monoxide.

**19** A process for the production of methanol from synthesis gas which process comprises reductively activating the catalyst precursor as produced by the process of claims 1 to 15 and contacting synthesis gas with the reductively activated catalyst at a temperature in the range from 80 to 300°C and a pressure of from 0 to 100 bar.

**20** A process according to claim 19 wherein the catalyst precursor has the formula (I) in which b = zero and reductive activation is effected with synthesis gas under methanol production conditions.

**21** A process according to claim 19 wherein the catalyst precursor has the formula (I) in which b is greater than zero and reductive activation is effected as a discrete step prior to methanol production using reducing gas substantially free from carbon monoxide.

**22** A process according to any one of claims 19 to 21 wherein synthesis gas is contacted with the reductively activated catalyst at a temperature in the range from 180 to 250°C.

23 A catalyst composition for use in the conversion of synthesis gas to methanol which composition comprises elemental copper and an oxide of thorium in the atomic ratio:

$$Cu_aThO_x$$

wherein x is a number such that the valence requirements of Th for oxygen is satisfied, and

a is in the range from 0.1 to 4,

and wherein at least 30% of the elemental copper particles are of a size less than or equal to 20 Angstroms.

**24** A composition according to claim 23 wherein at least 50% of the elemental copper particles are of a size less than or equal to 20 Angstroms.

**25** A composition according to claim 23 wherein at least 90% of the elemental copper particles are of a size less than or equal to 20 Angstoms.

**26** A composition according to any one of claims 23 to 25 wherein a in the formula (II) has a value in the range from 0.5 to 3.

**27** A composition according to claim 26 wherein a has a value in the range from 0.7 to 2.

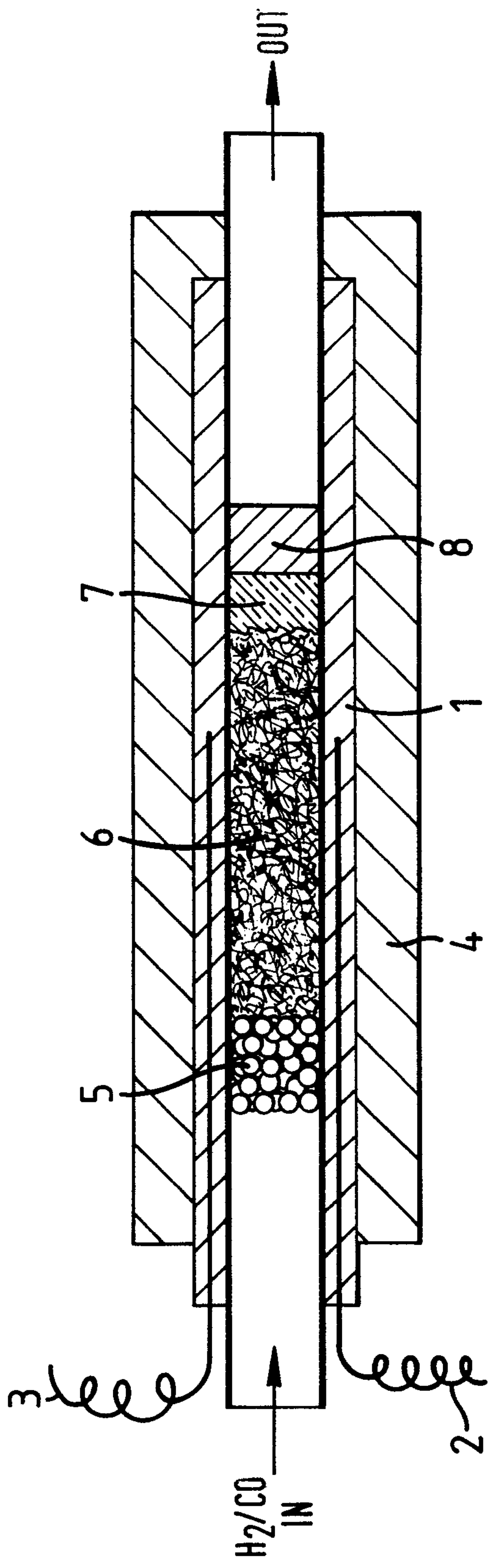


FIG.1

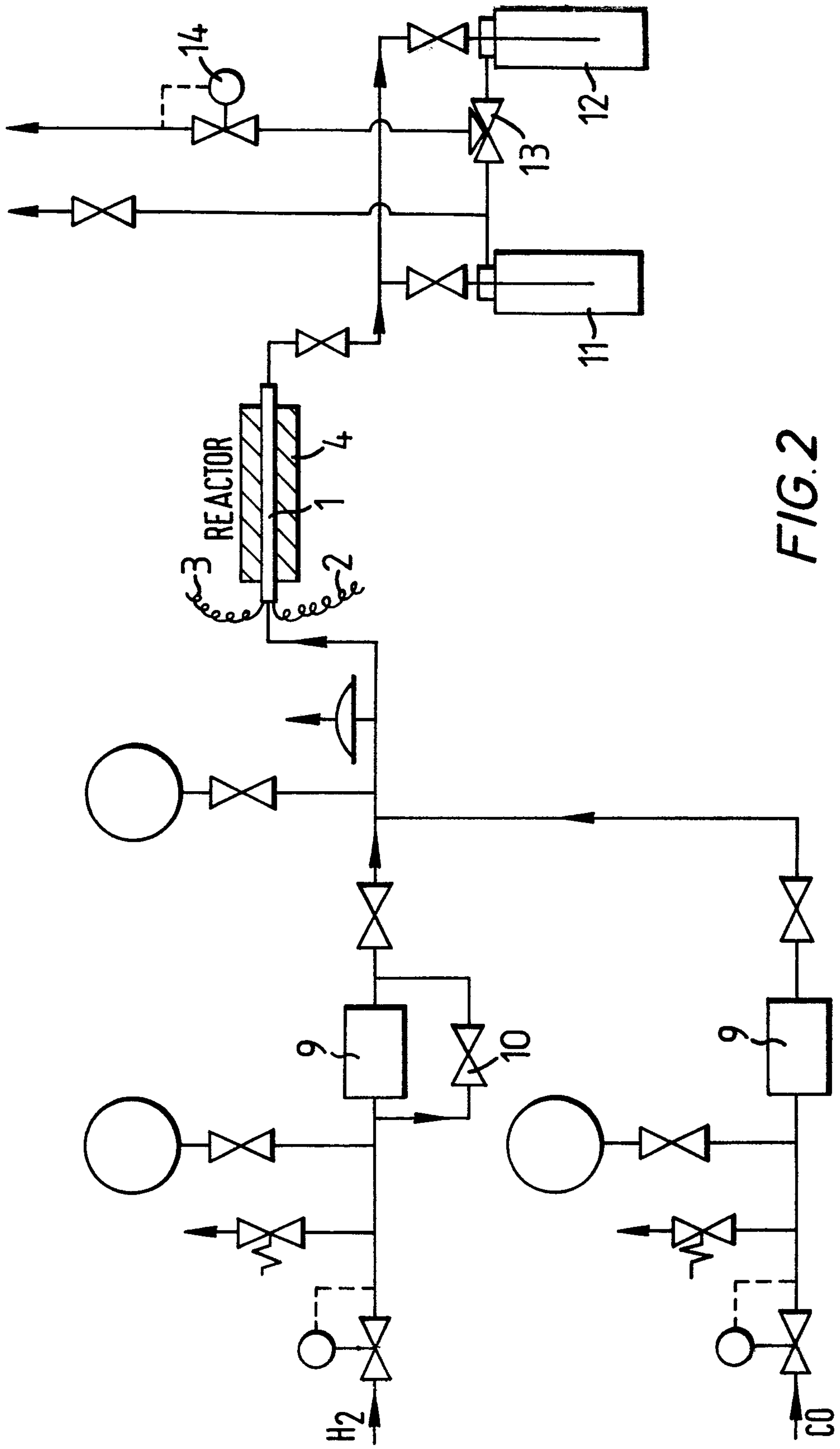


FIG. 2

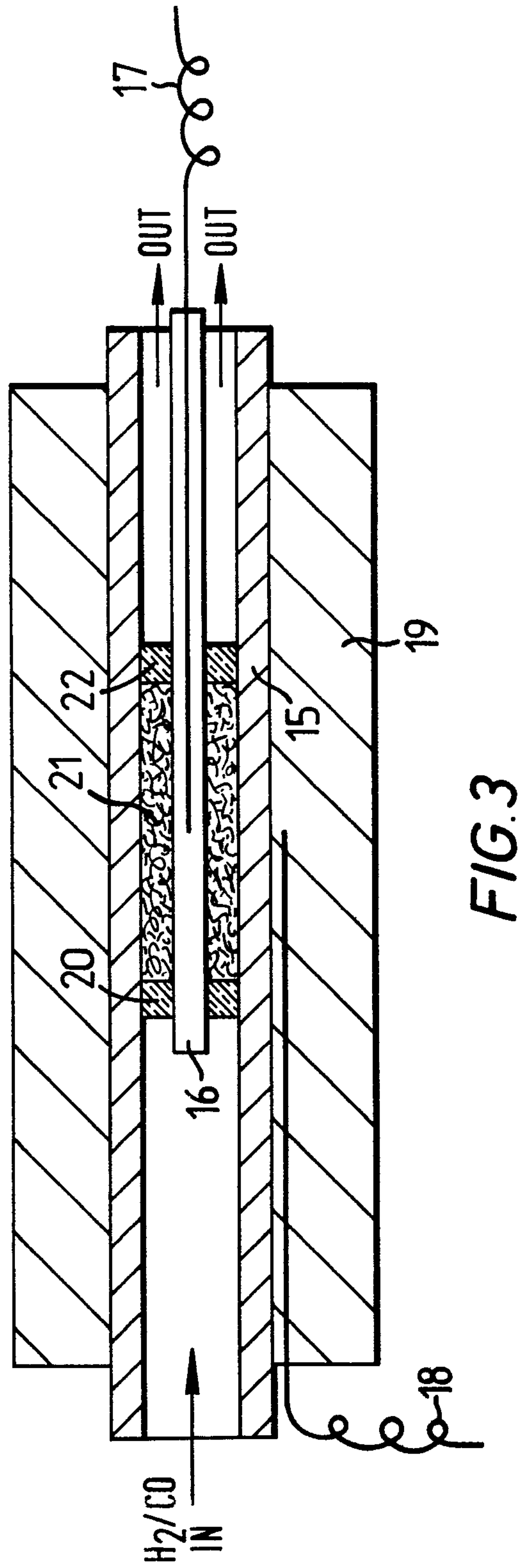


FIG.3

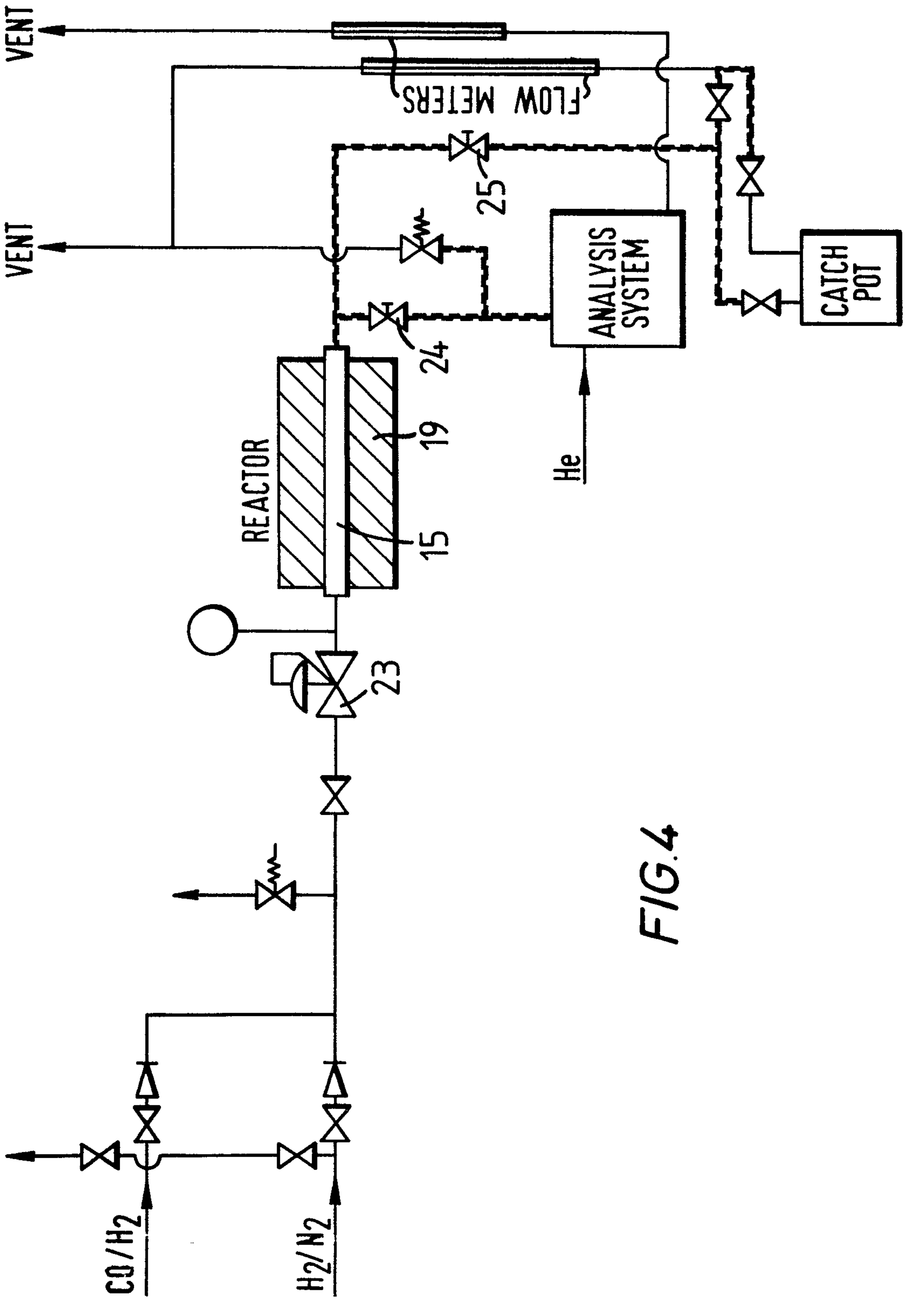
VENT
FLOW METERS
VENT
25
ANALYSIS SYSTEM
CATCH POT
24
REACTOR
19
15
He
23
CO/H2
H2/N2

FIG.4

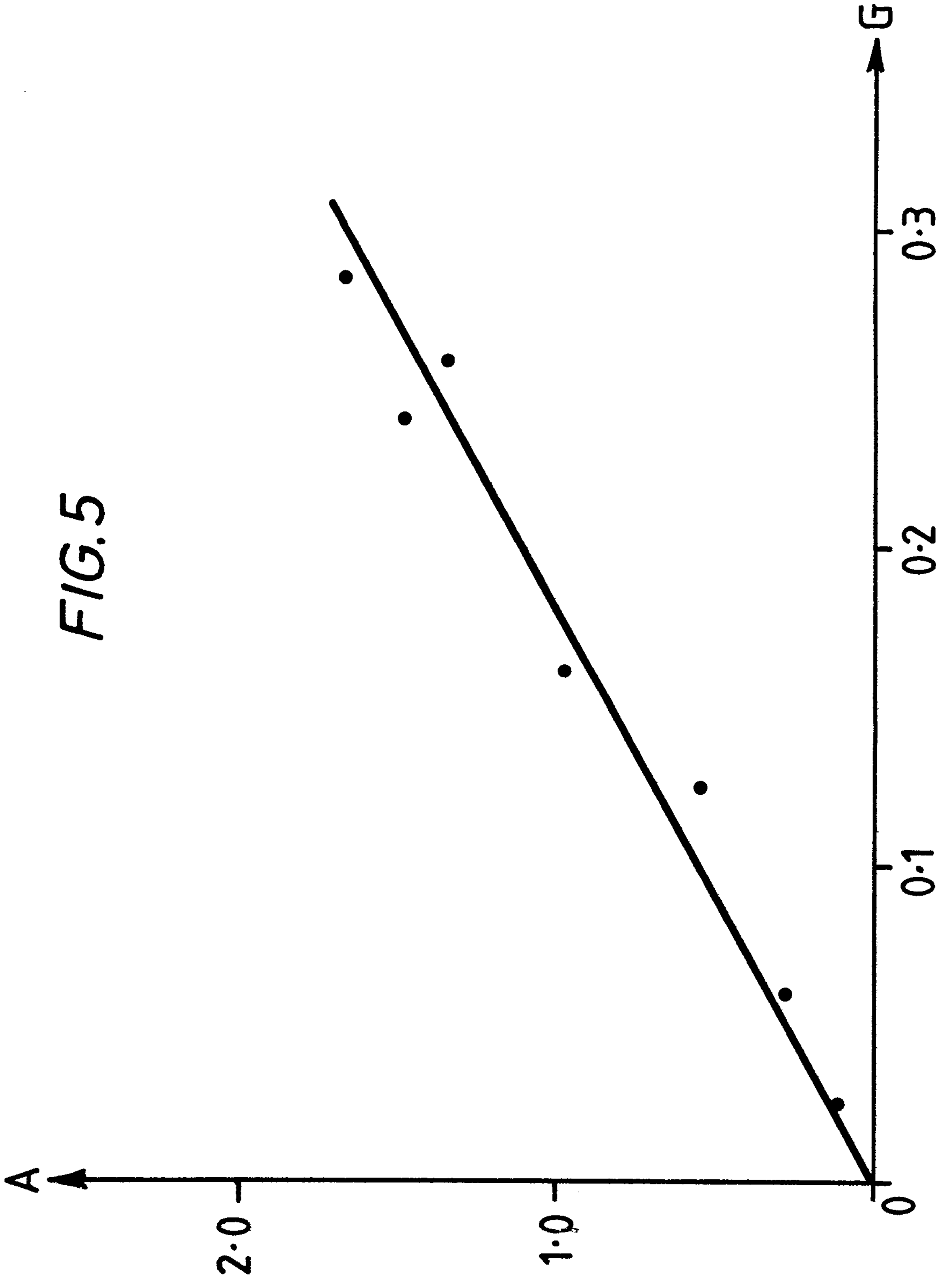


FIG.5

0171178

European Patent Office

# EUROPEAN SEARCH REPORT

Application number

EP 85 30 4703

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 005 492 (STANDARD OIL)<br><br>* Claims 1,7; example 7 *<br>--- | 1-3,7, 21,23, 26,27 | B 01 J 23/78<br>C 07 C 29/15 |
| X | EP-A-0 034 338 (SÜD CHEMIE AG)<br><br>* Claims *<br>--- | 1-3,7, 18-23, 26,27 | |
| A | FR-A-1 360 473 (THE DISTILLERS CO.)<br>----- | | |
| | | | **TECHNICAL FIELDS SEARCHED (Int. Cl.4)** |
| | | | B 01 J<br>C 07 C |

The present search report has been drawn up for all claims

| Place of search | Date of completion of the search | Examiner |
|---|---|---|
| THE HAGUE | 09-10-1985 | THION M.A. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82